(19) Europäisches Patentamt / European Patent Office / Office européen des brevets

(11) **EP 2 211 804 B1**

(12) **EUROPÄISCHE PATENTSCHRIFT**

(45) Veröffentlichungstag und Bekanntmachung des Hinweises auf die Patenterteilung:
**31.08.2016 Patentblatt 2016/35**

(51) Int Cl.:
*A61F 9/008* (2006.01)      *A61B 3/103* (2006.01)

(21) Anmeldenummer: **08847574.4**

(22) Anmeldetag: **03.11.2008**

(86) Internationale Anmeldenummer:
**PCT/EP2008/009247**

(87) Internationale Veröffentlichungsnummer:
**WO 2009/059730 (14.05.2009 Gazette 2009/20)**

(54) **BEHANDLUNGSVORRICHTUNG ZUR OPERATIVEN FEHLSICHTIGKEITSKORREKTUR EINES AUGES UND VERFAHREN ZUM ERZEUGEN VON STEUERDATEN DAFÜR**

TREATMENT DEVICE FOR OPERATIVELY CORRECTING DEFECTIVE VISION OF AN EYE AND METHOD FOR PRODUCING CONTROL DATA THEREFOR

DISPOSITIF DE TRAITEMENT PERMETTANT LA CORRECTION CHIRURGICALE DE L'AMÉTROPIE D'UN OEIL ET PROCÉDÉ DE GÉNÉRATION DE DONNÉES DE COMMANDE ASSOCIÉES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **08.11.2007 DE 102007053281**

(43) Veröffentlichungstag der Anmeldung:
**04.08.2010 Patentblatt 2010/31**

(73) Patentinhaber: **Carl Zeiss Meditec AG**
**07745 Jena (DE)**

(72) Erfinder:
• **BISCHOFF, Mark**
**07749 Jena (DE)**
• **STOBRAWA, Gregor**
**07743 Jena (DE)**
• **BISSMANN, Wilfried**
**07749 Jena (DE)**

(74) Vertreter: **Patentanwälte Geyer, Fehners & Partner mbB**
**Perhamerstrasse 31**
**80687 München (DE)**

(56) Entgegenhaltungen:
DE-A1-102005 014 760      DE-A1-102005 049 281
US-A- 6 110 166      US-A1- 2003 212 387
US-A1- 2004 243 112      US-A1- 2006 155 265
US-B1- 6 325 792

## Beschreibung

Die Erfindung bezieht sich auf eine Behandlungsvorrichtung zur operativen Fehlsichtigkeitskorrektur am Auge, wobei die Behandlungsvorrichtung eine von einer Steuereinrichtung gesteuerte Lasereinrichtung aufweist, welche durch Einstrahlen von Laserstrahlung Hornhaut-Gewebe trennt, wobei die Steuereinrichtung ausgebildet ist, die Lasereinrichtung zur Abgabe der Laserstrahlung in die Hornhaut so anzusteuern, dass damit ein lentikelförmiges Volumen in der Hornhaut isoliert ist, dessen Entfernung aus der Hornhaut die gewünschte Korrektur bewirkt.

Die Erfindung bezieht sich weiter auf ein Verfahren zum Erzeugen von Steuerdaten für eine Lasereinrichtung einer Behandlungsvorrichtung zur operativen Fehlsichtigkeitskorrektur am Auge, welche durch Einstrahlen von Laserstrahlung Hornhaut-Gewebe trennt, wobei die Steuerdaten im Betrieb die Lasereinrichtung zur Abgabe der Laserstrahlung in die Hornhaut so ansteuern, dass damit ein lentikelförmiges Volumen in der Hornhaut isoliert ist, dessen Entfernung aus der Hornhaut die gewünschte Korrektur bewirkt.

Solche Verfahren oder Vorrichtungen sind aus den US 2004/243112 A1 und US 6110166 A bekannt.

Der klassische Weg zur Korrektur der Fehlsichtigkeit des menschlichen Auges ist die Brille. Mittlerweile wird jedoch auch vermehrt refraktive Chirurgie eingesetzt, die durch Veränderung der Augenhornhaut eine Fehlsichtigkeitskorrektur bewirkt. Ziel der Operationsmethoden ist es dabei, die Hornhaut gezielt zu verändern, um so die Lichtbrechung zu beeinflussen. Hierfür sind unterschiedliche Operationsmethoden bekannt. Am verbreitesten ist gegenwärtig die sogenannte Laser-Insitu-Keratomileusis, die auch LASIK abgekürzt wird und z.B. in US 6325792 B1, US 2006/155265 A1, US 2003/212387 A1, DE 10 2005 014760 A1 oder DE 10 2005 049281 A1 beschrieben ist. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp. Irvine, USA, vertrieben wird. Nachdem die Lamelle gelöst und zur Seite geklappt wurde, ist bei der LASIK-Operation die Anwendung eines Excimer-Lasers vorgesehen, der das derart freigelegte Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt.

Die Anwendung eines Laserkeratoms zum Freilegen der Lamelle (auch "Flap" genannt) ist vorteilhaft, da die Infektionsgefahr dadurch verringert und die Schnittqualität vergrößert ist. Insbesondere kann die Lamelle mit sehr viel konstanterer Dicke hergestellt werden. Auch ist der Schnitt potentiell glatter, was spätere optische Störungen durch diese auch nach der Operation verbleibende Grenzfläche mindert.

Bei der Erzeugung einer Schnittfläche in der Hornhaut durch Laserstrahlung wird ins Gewebe üblicherweise gepulste Laserstrahlung eingebracht, wobei die Pulslänge in der Regel unter 1 ps liegt. Dadurch wird die zur Auslösung eines optischen Durchbruchs nötige Leistungsdichte für den jeweiligen Puls auf ein enges räumliches Gebiet begrenzt. Die US 5984916 zeigt diesbezüglich deutlich, dass der räumliche Bereich des optischen Durchbruches (in diesem Fall der erzeugten Wechselwirkung) stark von der Pulsdauer abhängt. Eine hohe Fokussierung des Laserstrahls in Kombination mit den erwähnten kurzen Pulsen erlaubt es damit, den optischen Durchbruch punktgenau in der Hornhaut einzusetzen. Zur Schnitterzeugung wird eine Serie optischer Durchbrüche an vorbestimmten Stellen so erzeugt, dass dadurch die Schnittfläche ausgebildet wird. Beim erwähnten Laserkeratom bildet die Schnittfläche die vor dem Einsatz der Laserablation abzuklappende Lamelle.

Bei der herkömmlichen LASIK-Methode wird freigelegtes Hornhautgewebe verdampft, was auch als "Schleifen" der Hornhaut mittels Laserstrahlung bezeichnet wird. Die Volumenentfernung, die für eine Fehlsichtigkeitskorrektur notwendig ist, wird dabei für jedes Flächenelement der freigelegten Hornhaut durch die Zahl der Laserpulse und deren Energie eingestellt. Je nach Zahl und Energie der Laserpulse erfolgt unterschiedlich viel Materialabtrag.

In jüngster Zeit wurde das eingangs erwähnte Operationsverfahren beschrieben und in ersten Erprobungen untersucht. Mittels Laserstrahlung wird ein Volumen in der Hornhaut isoliert und dann das dieses Volumen bildende Gewebestück entnommen. Da auch hier i.d.R. gepulste Laserstrahlung verwendet wird, spricht man von Femtosekemden-Lentikel-Extraktion oder kurz FLEx. Das Volumen wird als Lentikel bezeichnet.

Erfahrungswerte, die zum Schleifen der Hornhaut mittels Ablationslaserstrahlung tauglich sind, können nun für das FLEx-Verfahren der refraktiven Augenchirurgie, bei dem das aus der Hornhaut zu entfernende Volumen nicht durch Ablation freigelegten Hornhautgewebes abgetragen wird, sondern in der Hornhaut durch eine dreidimensionale Schnittfläche isoliert wird und somit entnehmbar gemacht, nicht verwendet werden, da die Ansätze, Verdampfen von zu entfernenden Material einerseits und Entnahme eines isolierten Volumens andererseits, zu unterschiedlich sind. Dies gilt ganz besonders für die Wahl der das zu entnehmenden Volumen begrenzenden Schnittfläche, da es solche bei der herkömmlichen LASIK-Operation gar nicht gibt. Auch sind die Heilungsprozesse nach der Operation andere, da die Flächen andere Oberflächenstrukturen haben.

Die manuelle Entfernung erfordert eine gewisse mechanische Stabilität des Gewebestücks, welche neben der beabsichtigten refraktiven Wirkung eine weitere Randbedingung darstellt. Zudem besteht der Wunsch nach einer Formgebung, die es erlaubt, die refraktiven Auswirkungen der Heilungsprozesse zu minimieren oder zumindest vorhersagbar zu gestalten.

Kommt es bei der manuellen Extraktion des isolierten Volumens zu dessen Zerstörung (Zerreißen), so wäre dies für die refraktive Wirkung noch kein Problem. Es besteht aber die Notwendigkeit, das isolierte Volumen möglichst vollständig zu entfernen, eine Bedingung die insbesondere in der optischen relevanten Zone (Projektion der geöffneten Pupille auf die Cornea) streng eingehalten werden sollte, um später optische Aberrationen zu vermeiden. Ein Zerreißen des isolierten Volumens wäre deshalb mit einem großen Zusatzaufwand bei der Entfernung eines Teils des isolierten Volumens verbunden, ist dadurch auch unsicherer und gefährdet den Erfolg einer refraktiven Therapie.

Es besteht weiter der Wunsch, refraktiven Veränderungen im Zuge der Heilungsprozesse (die sog. Regression), zumindest möglichst gering und vorhersagbar zu gestalten.

Insgesamt bestehen somit bei der refraktiven Fehlsichtigkeitskorrektur mittels FLEx in vielen Bereichen andere und z.T. auch neue Randbedingungen als bei der herkömmlichen LASIK-Methode.

Es ist deshalb Aufgabe der vorliegenden Erfindung, eine Behandlungsvorrichtung bzw. ein Verfahren der eingangs genannten Art so auszubilden, dass die Schnittflächen für das Lentikel sowohl für eine sichere Entnahme als auch für den Heilungsprozess günstig sind.

Diese Aufgabe wird erfindungsgemäß gelöst mit einer Behandlungsvorrichtung zur operativen Hyperopiekorrektur am Auge, wobei die Behandlungsvorrichtung eine von einer Steuereinrichtung gesteuerte Lasereinrichtung aufweist, welche durch Einstrahlen von Laserstrahlung Hornhaut-Gewebe trennt, wobei die Steuereinrichtung ausgebildet ist, die Lasereinrichtung zur Abgabe der Laserstrahlung in die Hornhaut so anzusteuern, dass damit ein lentikelförmiges Volumen in der Hornhaut isoliert ist, dessen Entfernung aus der Hornhaut die gewünschte Hyperopiekorrektur bewirkt, wobei das lentikelförmige Volumen eine posteriore Fläche und eine anteriore Fläche hat, deren Ränder über eine Randfläche verbunden sind, wobei die Schnittkurve aus der Randfläche und einer Ebene, in der die Sehachse enthalten ist, senkrecht zur Sehachse eine Breite hat, die größer als diejenige ist, welche in der gleichen Projektionsebene eine gerade Strecke hätte, die am Rand der posterioren oder der anterioren Fläche auf der jeweiligen Fläche senkrecht steht und die anteriore Fläche mit der posterioren Fläche oder mit deren gedachter Fortsetzung verbindet.

Diese Aufgabe wird weiter gelöst mit einem Verfahren zum Erzeugen von Steuerdaten für eine Lasereinrichtung einer Behandlungsvorrichtung zur operativen Hyperopiekorrektur am Auge, welche durch Einstrahlen von Laserstrahlung Hornhaut-Gewebe trennt, wobei die Steuerdaten im Betrieb die Lasereinrichtung zur Abgabe der Laserstrahlung in die Hornhaut so ansteuern, dass damit ein lentikelförmiges Volumen in der Hornhaut isoliert ist, dessen Entfernung aus der Hornhaut die gewünschte Hyperopiekorrektur bewirkt, wobei die Steuerdaten das lentikelförmige Volumen so vorgeben, dass es eine posteriore Fläche und eine anteriore Fläche hat, deren Ränder über eine Randfläche verbunden sind, wobei die Schnittkurve aus der Randfläche und einer Ebene, in der die Sehachse enthalten ist, senkrecht zur Sehachse eine Breite hat, die größer als diejenige ist, welche in der gleichen Projektionsebene eine gerade Strecke hätte, die am Rand der posterioren oder der anterioren Fläche auf der jeweiligen Fläche senkrecht steht und die anteriore Fläche mit der posterioren Fläche oder mit deren gedachter Fortsetzung verbindet.

Die Erfindung sorgt dafür, dass das zu entnehmende Volumen, also das in der Augenhornhaut isolierte Gewebestück ausreichende Stabilität hat, so dass ein Abreißen von Teilen des Gewebestückes beim Entfernen bzw. bei der Entnahme aus der Augenhornhaut mit ausreichender Sicherheit vermieden ist. Der Erfindung liegt dabei die Erkenntnis zugrunde, dass unterhalb einer gewissen Restdicke das Gewebe der Augenhornhaut zu fragil wird. Im Bereich dieser Restdicke wird erfindungsgemäß die Mindestdicke eingestellt. Bei einer Mindestdicke im Bereich von 5 bis 50 $\mu$m ist sichergestellt, dass das Hornhautgewebe nicht reißt und sich somit keine unerwünschten Partikel bilden, die beim Verbleiben in der Augenhornhaut eine schlechte optische Qualität zur Folge hätten.

Die Problematik der Mindestdicke stellt sich sowohl bei einer Hyperopie-Korrektur als auch bei der Korrektur von Myopie. Bei der Entnahme eines hyperopen Lentikels ist die Mindestdicke im Bereich der Sehachse, d.h. im zentralen Bereich des Lentikels gegeben. Bei einem myopen Lentikel wird dagegen die Mindestdicke am Rand vorgesehen.

Die Erfinder erkannten, dass es für die optische Gesamtqualität der Korrektur von Vorteil ist, mehr Volumen zu entnehmen, als an und für sich erforderlich ist, also eine Mindestdicke für das Lentikel vorzusehen. Dieser Widerspruch von an und für sich allen chirurgischen Eingriffen Innenwunden bestreben, möglichst sparsam mit Gewebe umzugehen, führt überraschenderweise zu einer besseren optischen Qualität, da keine Gewebefragmente in der Augenhornhaut zu befürchten sind. Der erfindungsgemäße Ansatz senkt deshalb den Bedarf für nachopparative Korrekturen drastisch.

Eine Weiterbildung der Erfindung verwendet die Mindestdicke zusätzlich dazu, um zu verhindern, dass postoperative (Regressions-)Probleme beim FLEX-Verfahren dadurch auftreten, dass die gelöste und wiederaufgelegte Hornhautlamelle sich am Rand des entnommenen Volumens ungünstig einfügt bzw. nicht glatt aufliegen kann. Zur Vermeidung solcher Regressionsprobleme schafft die Erfindung eine breite Randzone, so dass für die dort auf die posteriore Schnittfläche, die zur Isolierung des lentikelförmigen Volumens geschaffen wurde, übergehende Hornhautlamelle eine Übergangszone geschaffen ist, die so gut wie keine Regression zur Folge hat. Wenn diese Übergangszone vorzugsweise auch außerhalb des optisch wirksamen Bereichs liegt, also außerhalb der dunkeladaptierten Pupille des Auges, ist die Gefahr für weitere unerwünschte Nebenwirkungen nochmals verringert. Vorzugsweise hat die Übergangszone eine Breite zwischen 0,1 und 1 mm.

Es zeigte sich weiter, dass die Regressionen besonders gut gemindert sind, wenn die Randfläche möglichst senkrecht in die anteriore Fläche mündet. Ein solcher Verlauf ist primär überraschend, da bei einer solch senkrechten Mündung für die darüberliegende Hornhautlamelle auch eine senkrecht von der Hornhautvorderfläche wegführende Stufe gegeben sein kann. Diese senkrechte Mündung stellt zum einen die Mindestranddicke ein und erweist sich zum anderen hinsichtlich der Regression als unproblematisch, wenn die Randfläche unterhalb dieser senkrechten Mündung zusätzlich einen zweiten, stärker zur Sehachse hingeneigten Abschnitt aufweist. Durch diese Struktur hat das lentikelförmige Volumen eine Randdicke, die vorzugsweise zwischen 5 und 50 μm liegt. Es zeigte sich, dass eine solche Randdicke hinsichtlich der Regression unproblematisch ist, zugleich aber eine bessere Entnehmbarkeit des Gewebestückes gewährleistet, da durch die Randdicke mit ausreichender Sicherheit vermieden ist, dass beim Abnehmen des Gewebestückes Partikel am Rand abreißen. Solche Partikel beeinflussen das Auflegen der Hornhautlamelle nach Entnahme des Gewebes sehr viel stärker, als es die vergleichsweise geringer Randdicke des Gewebestückes und damit der kurze senkrecht mündende Abschnitt der Randfläche tut. Diese Ausgestaltung der Erfindung nimmt also eine a priori als negativ erscheinende Randstruktur und erzielt damit im Ergebnis eine geringere Regression, d.h. ein besseres Einwachsverhalten, als ein eigentlich zu erwartender möglichst schmaler Rand.

Der zweite Abschnitt der Randfläche, der stärker zur Sehachse hingeneigt ist, kann auf viele Arten ausgebildet werden. Beispielsweise ist ein gerader, in einem Winkel zum ersten Abschnitt stehender Verlauf möglich, der in einem Winkel von 80° bis 100° zur Richtung der Sehachse liegt. Der zweite Abschnitt der Randfläche kann dann als Anfasung verstanden werden. Es sind aber auch gekrümmte Verläufe möglich, beispielsweise ein bezogen auf den Durchtrittspunkt der Sehachse konkaver Verlauf, d.h. eine Krümmung des zweiten Abschnittes zur Sehachse hin. In diesem Fall ist der zweite Abschnitt dann beispielsweise in Form einer Abrundung ausgeführt.

Im Rahmen der Weiterbildung der Erfindung sind verschiedenste Geometrien für den Rand tauglich, die alle das Regressionsverhalten positiv beeinflussen, indem sie die geschilderte breite Übergangszone bereitstellen.

In einer Weiterbildung der Erfindung wird die Randstruktur mit einer Bemessungsregel für das Hornhautvolumen kombiniert, welche den Krümmungsradius definiert, den die Hornhaut nach der Entnahme des Volumens hat. Diese Fortbildung der Erfindung erlaubt also nicht nur eine regressionsoptimierte Randstruktur, sondern auch eine analytische Berechnung der posterioren und der anterioren Fläche.

Die Beschreibung der Hornhautvorderkrümmung nach der Korrektur geht dabei von Fehlsichtigkeitsdaten aus, welche die Brechkraft $B_{BR}$ einer für die Fehlsichtigkeitskorrektur tauglichen Brille angeben, die in einem Abstand $d_{HS}$ vor dem Hornhautscheitel liegen muss, um die gewünschte Fehlsichtigkeitskorrektur zu erreichen. Das Bestimmen dieser Parameter ist gängiger Standard in der Augenheilkunde und ermöglicht die Verwendung vorhandener Messgeräte. Selbstverständlich können die dabei verwendeten Messdaten auch Astigmatismusfehler oder Fehler höherer Aberrationsordnungen wiedergeben, so dass die Gleichung zur Beschreibung des Krümmungsradius, der um das Volumen verminderten Hornhaut dann entsprechende Winkelparameter (bezogen auf zylindrische Koordinaten) hat, wie nachfolgende Gleichung (1) der Figurenbeschreibung wiedergibt.

Es ist deshalb eine Weiterbildung der erfindungsgemäßen Vorrichtung bzw. des erfindungsgemäßen Verfahrens bevorzugt, bei der die anteriore Fläche in einem konstantem Abstand $d_F$ zur Hornhautvorderfläche liegt und die posteriore Fläche gekrümmt ist und einen Krümmungsradius $R_L = R_{CV}{}^* - d_F$ hat, wobei $R_{CV}{}^*$ folgender Gleichung genügt $R_{CV}{}^* = 1 / ( ( 1/R_{CV}) + B_{BR} / ((n_c-1) \cdot (1 - d_{HS} \cdot B_{BR}))) + F$, und $R_{CV}$ der Krümmungsradius der Hornhaut vor Entfernung des Volumens, $n_c$ die Brechkraft des Materials der Hornhaut, F ein Korrektur-Faktor ist, $B_{BR}$ die Brechkraft einer für die Fehlsichtigkeitskorrektur tauglichen Brille, sowie $d_{HS}$ der Abstand ist, in dem die Brille mit der Brechkraft $B_{BR}$ vor dem Hornhautscheitel liegen müßte, um die gewünschte Fehlsichtigkeitskorrektur mittels der Brille zu erreichen.

Der Korrektur-Faktor F stellt ein Maß für die optische Wirkung der Dickenabnahme der Augenhornhaut auf der Sehachse dar, welche sich durch die Entfernung des Volumens ergibt. In einer vereinfachten Berechnung kann der Faktor F=0 gesetzt werden. In einer genaueren Berechnung kann F wie folgt berechnet werden: $F = (1 - 1/n_c) \cdot (d_C{}^* - d_C)$, wobei $d_C$ bzw. $d_C{}^*$ die Dicke der Hornhaut vor bzw. nach Entfernung des Volumens bezeichnet und der Radius $R_{CV}{}^*$ iterativ berechenbar ist, indem bei jedem Iterationsschritt aus der Differenz $(RCV^* - Rcv)$ auf eine Dickenänderung $(d_C{}^* - d_C)$ geschlossen wird und das entsprechende daraus erhaltene Ergebnis für die Dickenänderung bei der Berechnung von $R_{CV}{}^*$ im nächsten Iterationsschritt angewendet wird. Die iterative Berechnung für F kann beispielsweise abgebrochen werden, wenn zwischen zwei Iterationsschritten für F nur noch ein Unterschied besteht, der kleiner als ein bestimmter Grenzwert ist.

Die in der Weiterbildung vorgesehene Ausgestaltung der posterioren Fläche mit einer Krümmung, welche auf die der Hornhautvorderfläche nach der Entfernung des Volumens Bezug nimmt, erlaubt eine besonders einfache Definition der das Volumen begrenzenden Flächen, da die anteriore Fläche nun in einem konstantem Abstand zur Hornhautvorderfläche liegt und die optische Korrektur durch die Formgebung der posterioren Fläche bewirkt wird. Merklicher Rechenaufwand entsteht dann nur noch für die Definition der posterioren Teilfläche, nicht hingegen für die anteriore Teilfläche. Weiter zeigt sich auch, dass bei einem derartigen Ansatz zugleich eine einfache analytische Beschreibung der posterioren Teilfläche möglich ist.

Das erfindungsgemäße Verfahren zum Vorbereiten der Steuerdaten kann ohne menschliche Mitwirkung durchgeführt werden. Insbesondere kann es von einem Computer ausgeführt werden, der aus entsprechenden Vorgaben, beispielsweise aus Messdaten des Auges die Steuerdaten ermittelt. Vor allem ist bei der Ermittlung der Steuerdaten die Mitwirkung eines Arztes in keiner Weise erforderlich, da mit der Ermittlung der Steuerdaten noch kein therapeutischer Eingriff verbunden ist. Dieser findet erst bei der Anwendung der zuvor ermittelten Steuerdaten statt.

Soweit in dieser Beschreibung Verfahrensschritte geschildert werden, ist in der erfindungsgemäßen Vorrichtung ein Steuergerät vorgesehen, dass für die Ausführung der Verfahrensschritte beim Betrieb der Vorrichtung Sorge trägt.

Die Erfindung wird nachfolgend unter Bezugnahme auf die Zeichnungen beispielshalber noch näher erläutert. In den Zeichnungen zeigt:

Fig. 1        eine Schemadarstellung einer Behandlungsvorrichtung bzw. eines Behandlungsgerätes zur Fehlsichtigkeitskorrektur,

Fig. 1a       eine Schemadarstellung hinsichtlich des Aufbaus des Behandlungsgerätes der Fig. 1,

Fig. 2        eine Prinzipdarstellung zur Einbringung gepulster Laserstrahlung in das Auge bei der Fehlsichtigkeitskorrektur mit dem Behandlungsgerät der Fig. 1,

Fig. 3        eine weitere Schemadarstellung des Behandlungsgerätes der Fig. 1,

Fig. 4        in Teilfiguren (a), (b) und (c) schematische Schnittdarstellungen zur Verdeutlichung des Korrekturbedarfes am menschlichen Auge bei Fehlsichtigkeit,

Fig. 5        eine schematische Schnittdarstellung durch die Augenhornhaut mit Darstellung eines zur Fehlsichtigkeitskorrektur zu entfernenden Volumens,

Fig. 6        ein Schnitt durch die Augenhornhaut nach Entfernung des Volumens der Fig. 5,

Fig. 7        eine Schnittdarstellung ähnlich der Fig. 5,

Fig. 8        eine schematische Schnittdarstellung durch die Augenhornhaut zur Veranschaulichung der Volumenentnahme,

Fig. 9a       eine Draufsicht auf die Augenhornhaut zur Veranschaulichung der bei der Fehlsichtigkeitskorrektur erzeugten Schnittflächen,

Fig. 9b       eine Schnittdarstellung zur Draufsicht der Fig. 9a, in der das Profil eines zur Hyperopie-Korrektur entfernten Volumens veranschaulicht ist,

Fig. 10a      eine Darstellung ähnlich der Fig. 9a, hier jedoch für eine Myopie-Korrektur,

Fig. 10b      eine Darstellung ähnlich der Fig. 9a, jedoch wiederum für Myopie-Korrektur,

Fig. 11 a     eine Darstellung ähnlich der Fig. 9a, jedoch mit einer anderen Schnittführung bezüglich der Entnahme des Volumens,

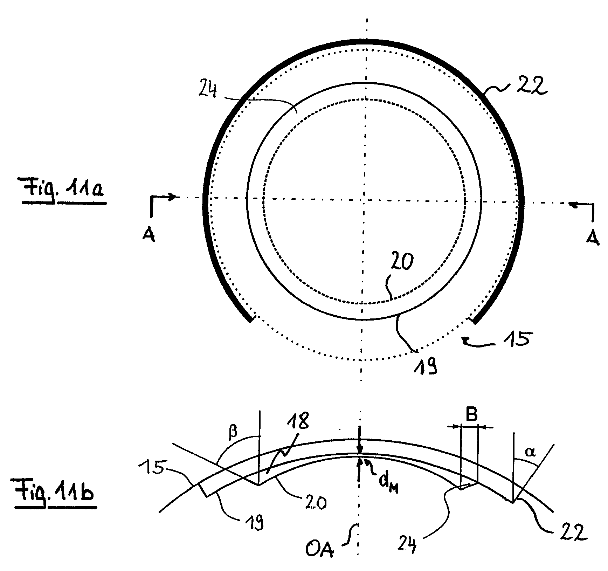

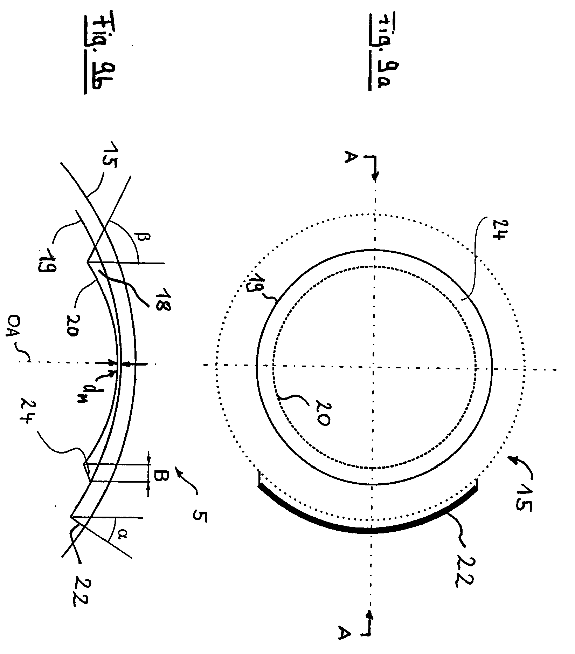

Fig. 11       b eine Schnittdarstellung ähnlich der Fig. 9b, jedoch für die Draufsicht der Fig. 11a,

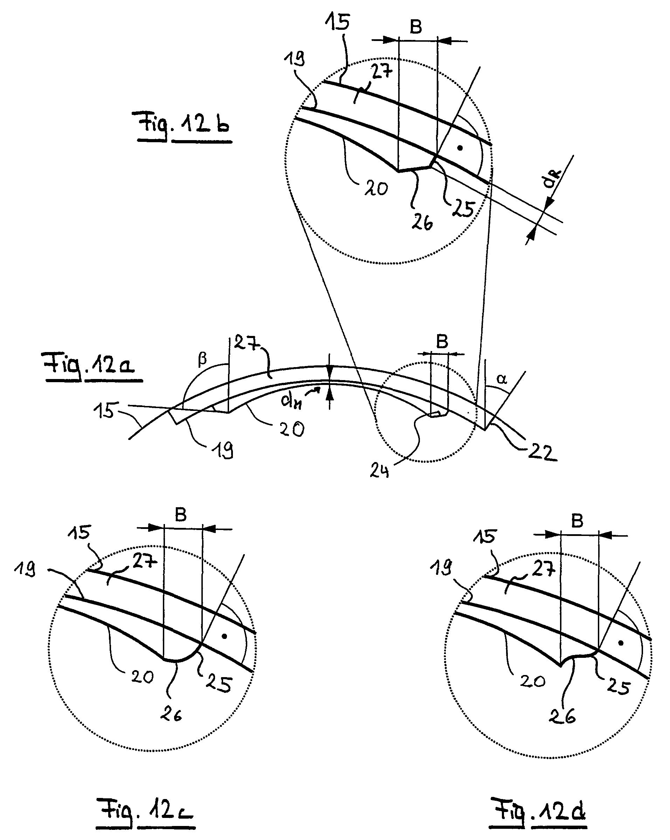

Fig. 12a      eine Schnittdarstellung ähnlich der Fig. 9a, jedoch mit einem andersartigen Randschnitt zur Begrenzung des Volumens, welches zur Fehlsichtigkeitskorrektur entnommen wird,

Fig. 12b      eine vergrößerte Teilansicht des Randschnittes der Fig. 9a und

Fig. 12c      und 12d eine vergrößerte Randschnitt-Darstellungen ähnlich der Fig. 12b, jedoch für andere Geometrien der Randfläche.

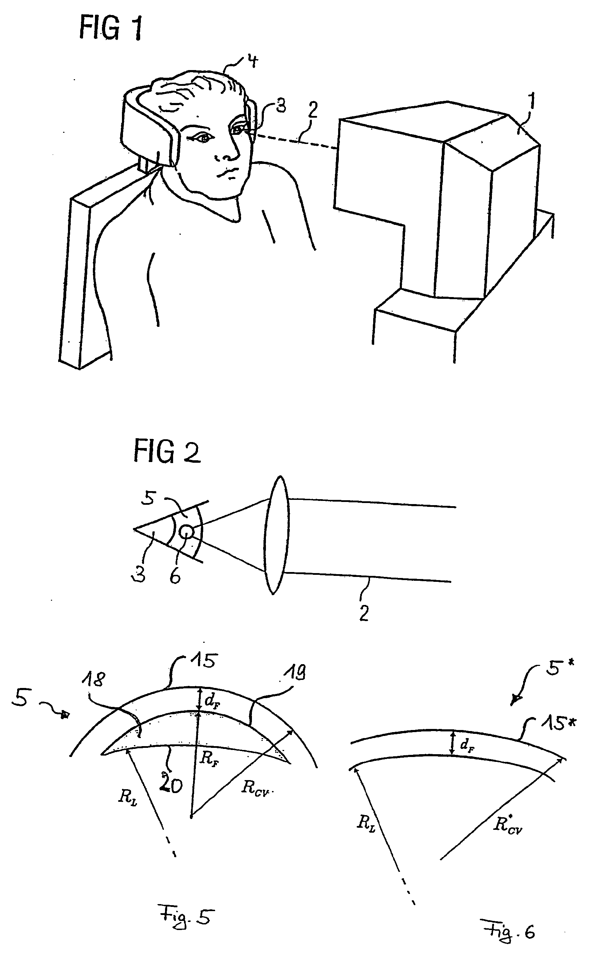

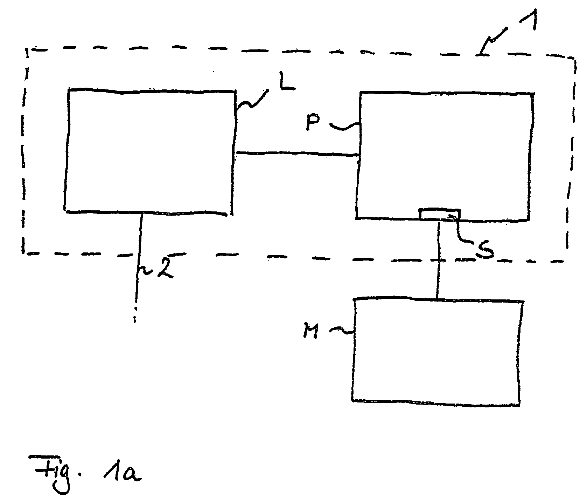

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Abberationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Messeinrichtungen vermessen.

Figur 1a zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die für den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, dass weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Messdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Messeinrichtung M, die das Auge des Patienten 4 zuvor vermessen hat. Natürlich kann die Messeinrichtung M auf beliebige Art und Weise die entsprechenden Mess- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Messeinrichtung M mit der Behandlungseinrichtung 1 hinsichtlich der Datenübertragung, die in einer Variante verwendet werden kann, hat den Vorteil, dass die Verwendung falscher Mess- und Fehlsichtigkeitsdaten mit größtmöglicher Sicherheit ausgeschlossen ist. Dies gilt insbesondere dann, wenn die Überführung des Patienten von der Messeinrichtung M bzw. den Messeinrichtungen zur Lasereinrichtung L mittels einer (in der Figur nicht dargestellten) Lagerungseinrichtung erfolgt, die mit der Messeinrichtung M bzw. der Lasereinrichtung L so zusammenwirkt, dass die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Messeinrichtung M zur Lasereinrichtung L kann dabei zugleich auch die Übertragung der Mess- und Fehlsichtigkeitsdaten an die Behandlungsvorrichtung 1 erfolgen.

Es ist vorzugsweise durch geeignete Mittel sichergestellt, dass die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

Die Wirkungsweise des Laserstrahls 2 ist in Figur 2 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus, der einen Spot 6 überdeckt und in dem die Laserstrahlungsenergiedichte so hoch ist, dass in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 2 schematisch angedeutete Plasmablase initiiert. Dadurch wird mittels dieses Laserpulses in der Hornhaut 5 Gewebe getrennt. Bei Entstehung einer Plasmablase umfasst die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch von jedem Laserpuls erzeugt wird, muss die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, dass mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, wesentlich ist lediglich, dass dazu gepulste Behandlungs-Laserstrahlung 2 verwendet wird. Beispielsweise kann ein Behandlungsgerät 1 verwendet werden, wie sie in der WO 2004/032810 A2 beschrieben ist. Wesentlich ist weiter, dass eine Vielzahl von Laserpulsfoki im Gewebe eine Schnittfläche ausbildet, deren Form vom Muster abhängt, mit dem die Laserpulsfoki im Gewebe angeordnet sind/werden. Das Muster gibt Zielpunkte für die Fokuslage vor, an denen ein oder mehrere Laserpuls(e) abgegeben wird(werden), und definiert die Form und Lage der Schnittfläche. Für die nachfolgend erläuterten Verfahren und Vorrichtungen ist das Muster der Zielpunkte von Bedeutung und wird noch näher beschrieben werden.

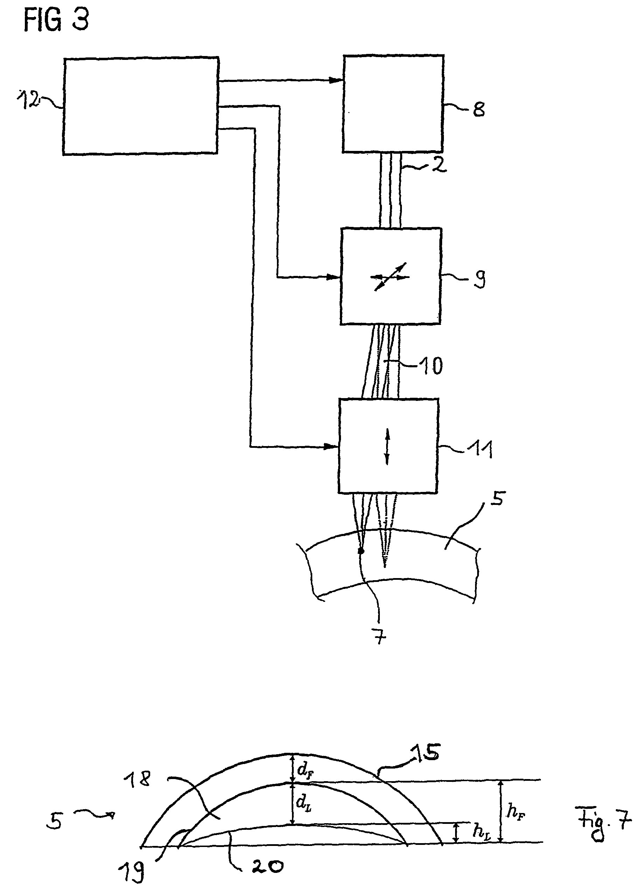

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, dass damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemet'schen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

In Figur 3 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so dass zur Schnittflächenerzeugung an verschiedenen Stellen fokussiert Energie aus Laserstrahlungspulsen in das Gewebe der Hornhaut 5 eingetragen wird. Die Laserstrahlung 2 wird von einem Laser 8 als gepulste Strahlung bereitgestellt. Ein xy-Scanner 9, der in einer Variante durch zwei im wesentlichen orthogonal ablenkende Galvanometerspiegel realisiert ist, lenkt den vom Laser 8 kommenden Laserstrahl zweidimensional ab, so dass nach dem xy-Scanner 9 ein abgelenkter Laserstrahl 10 vorliegt. Der xy-Scanner 9 bewirkt somit eine Verstellung der Lage des Fokus 7 im wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, dass die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, dass eine dreidimensionale Beschreibung der Lage des Fokus 7 in der Hornhaut 5 auch durch andere Koordinatensysteme erfolgen kann, insbesondere muss es sich nicht um ein rechtwinkliges Koordinatensystem handeln. Dass der xy-Scanner 9 um zueinander rechtwinklige Achsen ablenkt ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-geradlinige Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 3 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so dass die Lage des Fokus 7 in der Hornhaut 5 so verstellt wird, dass letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefasst. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muss eine Vorplanung des operativen Eingriffes dahingehend erfolgen, dass die Steuerdaten für das Behandlungsgerät 1 ermittelt werden, deren Anwendung dann eine für den Patienten 4 optimale Fehlsichtigkeitskorrektur erreicht.

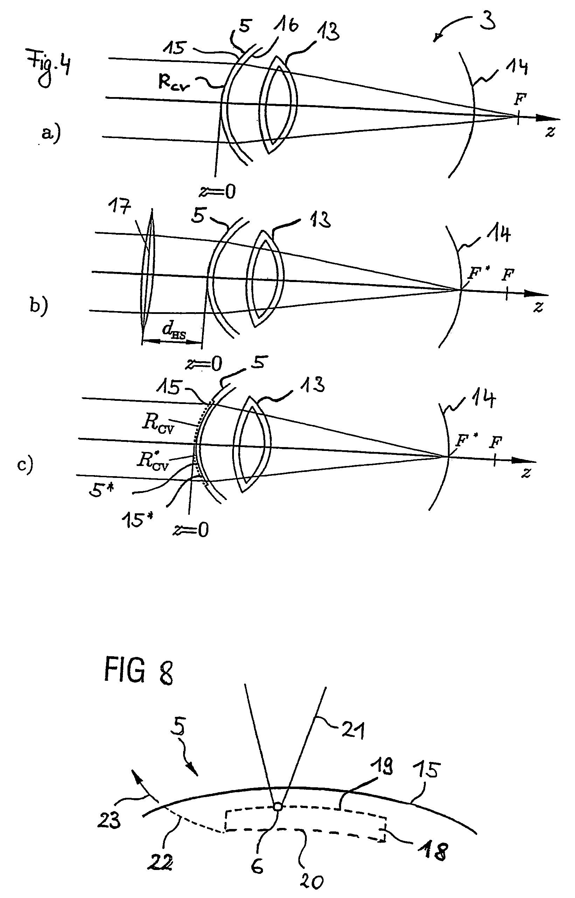

Zuerst gilt es das in der Hornhaut 5 zu isolierende und später zu entfernende Volumen festzulegen. Wie bereits anhand Fig. 1a geschildert bedarf es dazu einer Feststellung des Korrekturbedarfs. Figur 4 zeigt in Teilfiguren a), b) und c) die optischen Verhältnisse am Auge 3 des Patienten 4. Ohne Fehlsichtigkeitskorrektur liegt die in Teilfigur a) gezeigte Situation vor. Die Hornhaut 5 bewirkt zusammen mit der Augenlinse 13 eine Fokussierung eines im Unendlichen liegenden Gegenstandes in einen Fokus F, der auf der z-Achse hinter der Netzhaut 14 liegt. Die abbildende Wirkung rührt dabei zum einen von der bei nichtakkomodiertem Auge entspannten Augenlinse 13 sowie zum anderen von der Augenhornhaut 5 her, die im wesentlichen durch eine Hornhautvorderfläche 15 sowie eine Hornhautrückseite 16 definiert ist und aufgrund ihrer Krümmung ebenfalls eine abbildende Wirkung hat. Die optische Wirkung der Hornhaut 5 ist durch den Krümmungsradius Rcv der Hornhautvorderfläche bedingt. Teilfigur a) stellt die Fehlsichtigkeit nur exemplarisch dar, real können die oben erwähnten komplexeren Fehlsichtigkeiten vorliegen. Für sie gilt die nachfolgenden Beschreibung jedoch ebenfalls, allerdings können die angegebenen Gleichungen dann mitunter eine zusätzliche Winkelabhängigkeit beinhalten, auch wenn darauf nicht ausdrücklich hingewiesen wird.

Zur Fehlsichtigkeitskorrektur wird bekannter Weise, wie in Teilfigur b) der Figur 4 dargestellt, eine Vorsatz-Linse 17 in Form einer Brille im Abstand $d_{HS}$ vom Scheitelpunkt der Hornhaut 5 vor das Auge 3 gesetzt. Die Linse 17 der Brille ist in ihrer Brechkraft $B_{BR}$ so angepasst, dass sie den Fernpunkt des gesamten Systems, d.h. aus Brille und Auge, vom Fokuspunkt F zum korrigierten Fokuspunkt F* verschiebt, der auf der Netzhaut 14 liegt.

Hinsichtlich der in dieser Beschreibung verwendeten Nomenklatur sei angemerkt, dass durch die Anfügung eines Sterns an Größen verdeutlicht wird, dass es sich um Größen handelt, die nach einer Korrektur erhalten werden. Der Fokus F* ist also derjenige Fokus, der nach der optischen Korrektur vorliegt, die in der Teilfigur b) der Figur 4 durch die Linse 17 der Brille erreicht wird.

Unter der gerechtfertigten Annahme, dass eine Dickenänderung der Hornhaut 5 im wesentlichen den Krümmungsradius der Luft zugewandten Hornhaut-Vorderseite 15 modifiziert, nicht aber den Krümmungsradius der dem Augeninneren zuliegenden Hornhautrückseite 16, wird durch die Volumenentfernung der Krümmungsradius Rcv der Hornhautvorderseite 15 modifiziert. Die um das Volumen verminderte Hornhaut 5 hat eine derart geänderte Abbildungswirkung, dass der dann korrigierte Fokus F* auf der Netzhaut 14 liegt. Nach der Korrektur liegt eine veränderte Hornhautvorderfläche 15* vor, und es ist eine Fehlsichtigkeitskorrektur auch ohne Brille erreicht.

Zur Bestimmung des Musters der Zielpunkte wird deshalb die zu erreichende Krümmung der modifizierten Hornhautvorderfläche 15* ermittelt. Dabei ist Ausgangspunkt die Brechkraft der Linse 17 der Brille, da die Ermittlung der entsprechenden Parameter ein Standardverfahren in der Augenoptik ist. Für die Brechkraft $B_{BR}(\varphi)$ der Linse 17 der Brille gilt folgende Formel:

$$(1) \qquad B_{BR}(\varphi) = Sph + Cyl \cdot \sin^2(\varphi - \theta).$$

In dieser Gleichung bezeichnen Sph und Cyl die zu realisierenden Korrekturwerte sphärischen bzw. astigmatischen Brechungsfehler und $\theta$ die Lage der Zylinderachse der zylindrischen (astigmatischen) Fehlsichtigkeit, wie sie dem Fachmann in der Optometrie bekannt sind. Der Parameter $\varphi$ schließlich bezieht sich auf ein Zylinderkoordinatensystem des Auges und wird auf das Auge schauend entgegen dem Uhrzeigersinn gezählt, wie es in der Augenoptik üblich ist. Mit dem Wert $B_{BR}$ wird nun die Krümmung der modifizierten Hornhautvorderfläche 15* wie folgt eingestellt:

$$(2) \qquad R_{CV}{}^* = 1 / ( (1/R_{CV}) + B_{BR} / ( (n_c\text{-}1) \cdot (1 - d_{HS} \cdot B_{BR}))) + F$$

In Gleichung (2) bezeichnet $n_c$ die Brechkraft des Materials der Hornhaut. Der entsprechende Wert liegt üblicherweise bei 1,376; $d_{HS}$ bezeichnet den Abstand, in dem eine Brille mit der Brechkraft $B_{BR}$ vom Hornhautscheitel liegen muss, um die gewünschte Fehlsichtigkeitskorrektur mittels Brille zu erzeugen; $B_{BR}$ bezeichnet die zuvor erwähnte Brechkraft der Brille gemäß Gleichung (1). Die Angabe für die Brechkraft $B_{BR}$ kann auch Fehlsichtigkeiten erfassen, die über eine normale sphärische oder zylindrische Korrektur hinausgehen. $B_{BR}$ (und damit automatisch auch $R_{CV}{}^*$) haben dann zusätzliche Koordinatenabhängigkeiten.

Der Korrektur-Faktor F berücksichtigt die optische Wirkung der Dickenänderung der Hornhaut aus und kann in erster Näherung als konstanter Faktor angesehen werden. Für eine hochgenaue Korrektur kann der Faktor gemäß folgender Gleichung errechnet werden:

$$(3) \qquad F = (1 - 1/n_c) \cdot (d_C{}^* - d_C).$$

$d_C$ bzw. $d_C{}^*$ ist dabei die Hornhautdicke vor bzw. nach der optischen Korrektur. Für eine genaue Bestimmung erfolgt eine Berechnung von $R_{CV}{}^*$ iterativ, indem bei der i-ten Berechnung aus der Differenz $(R_{CV}{}^* - R_{CV})$ auf die Größe $(d_C{}^* - d_C)$ geschlossen wird und das entsprechende daraus erhaltene Ergebnis für die Dickenänderung bei der (i+1)-ten Berechnung angewendet wird. Dies kann man so lange durchführen, bis ein Abbruchkriterium erfüllt wird, beispielsweise wenn die Differenz des Ergebnisses für die Dickenänderung bei zwei aufeinanderfolgenden Iterationsschritten unter einer entsprechend festgelegten Grenze liegt. Diese Grenze kann beispielsweise über eine konstante Differenz festgelegt werden, die einer für die Behandlung angemessene Genauigkeit der Refraktionskorrektur entspricht.

Vernachlässigt man die Dickenänderung der Augenhornhaut, was für ein vereinfachtes Verfahren durchaus zulässig ist, kann der Korrektur-Faktor F in Gleichung (2) für eine vereinfachte Berechnung auch gleich Null gesetzt, also vernachlässigt und weggelassen werden. Man erhält überraschenderweise folgende einfache Gleichung für die Brechkraft der modifizierten Hornhaut 5*:

$$B_{CV}^* = B_{CV} + B_{BR} / (1 - B_{BR} \cdot d_{HS})$$

Aus dieser Gleichung ergibt sich für den Fachmann auf einfache Art und Weise mittels der Gleichung $B_{CV}^* = (n-1) / R_{CV}^*$ der Radius $R_{CV}^*$ der Hornhautvorderfläche 15*, der nach der Modifikation vorliegen muss, um die gewünschte Fehlsichtigkeitskorrektur zu erhalten, zu:

$$R_{CV}^* = 1 / ( (1/R_{CV}) + B_{BR} / ( (n_c-1) \cdot (1 - d_{HS} \cdot B_{BR}))).$$

Für das Volumen, dessen Entfernung die obige Krümmungsänderung der Hornhautvorderfläche 15 bewirkt, wird nun die das Volumen isolierende Grenzfläche festgelegt. Dabei ist vorzugsweise zu berücksichtigen, dass sich der Durchmesser des zu korrigierenden Bereichs und damit der Durchmesser des zu entnehmenden Volumens möglichst über die Pupillengröße bei dunkelangepasstem Auge erstrecken sollte.

In einer ersten Variante wird mittels dem Fachmann bekannter numerischer Methoden eine Freifläche definiert werden, die ein Volumen umschreibt, dessen Entfernung die Krümmungsänderung bewirkt. Dazu wird entlang der z-Achse die Dickenänderung ermittelt, die zur gewünschten Krümmungsmodifikation nötig ist. Daraus ergibt sich das Volumen als Funktion von r, $\varphi$ (in Zylinderkoordinaten) und daraus wiederum dessen Grenzfläche.

Eine einfache analytische Rechnung liefert die folgende zweite Variante, bei der die Grenzfläche des Volumens durch zwei Teilflächen aufgebaut wird, eine zur Hornhautoberfläche 15 hinliegende anteriore Teilfläche und eine gegenüberliegende posteriore Teilfläche. Die entsprechenden Verhältnisse zeigt Figur 5. Das Volumen 18 ist zur Hornhautvorderfläche 15 hin durch eine anteriore Schnittfläche 19 begrenzt, die in konstantem Abstand $d_F$ unter der Hornhautvorderfläche 15 liegt. Diese anteriore Schnittfläche 19 wird in Analogie zur Laserkeratomen auch als Flap-Fläche 19 bezeichnet, da sie dort dazu dient, in Kombination mit einem Öffnungsschnitt zum Rand hin die Augenhornhaut 5 eine Lamelle in Form eines "Flap" von der darunterliegenden Hornhaut 5 abheben zu können. Diese Art der Entnahme des zuvor isolierten Volumens 18 ist natürlich auch hier möglich.

Die anteriore Schnittfläche 19 hat einen Krümmungsverlauf, der um $d_F$ unter der Hornhautvorderfläche 15 liegt. Ist diese sphärisch, kann für die Flap-Fläche 19 ein Krümmungsradius angegeben werden, der um $d_F$ geringer ist als der Krümmungsradius $R_{CV}$. Wie später für bevorzugte Varianten beschrieben wird, kann bei der Erzeugung der Schnittfläche 19 durch ein Kontaktglas dafür gesorgt werden, dass die Hornhautvorderfläche 15 zum Zeitpunkt der Schnittflächenerzeugung sphärisch ist, so dass das Muster der Zielpunkte eine sphärische Schnittfläche bewirkt. Die Relaxation des Auges 3 nach Abnahme des Kontaktglases mag dann zwar zu einer nicht-sphärischen Schnittfläche 19 führen, sie hat aber dennoch konstanten Abstand zur Hornhautvorderfläche 15 bzw. 15*.

Posterior ist das Volumen 18, das aus der Hornhaut 5 entfernt werden soll, durch eine posteriore Schnittfläche 20 begrenzt, die im Allgemeinen keinen konstantem Abstand zur Hornhautvorderfläche 15 hat. Die posteriore Schnittfläche 20 wird deshalb so ausgebildet sein, dass das Volumen 18 in Form eines Lentikels vorliegt, weshalb die posteriore Schnittfläche 20 auch als Lentikel-Fläche 20 bezeichnet wird. In Figur 5 ist sie exemplarisch für eine Myopiekorrektur als ebenfalls sphärische Fläche mit einem Krümmungsradius $R_L$ eingezeichnet, wobei im Allgemeinen das Zentrum dieser Krümmung nicht mit dem Krümmungszentrum der in Figur 5 ebenfalls sphärischen Hornhautvorderfläche 15 zusammenfällt. Bei einer Hyperopiekorrektur ist $R_L$ größer als $R_{CV}$ - $d_F$.

Figur 6 zeigt die Verhältnisse nach Entfernung des Volumens 18. Der Radius der modifizierten Hornhautvorderfläche 15* beträgt nun $R_{CV}^*$ und kann beispielsweise gemäß den zuvor beschriebenen Gleichungen berechnet werden. Die zentrale Dicke $d_L$ des entnommenen Volumens 18 ist dabei maßgeblich für die Radiusänderung, wie Figur 7 verdeutlicht. In dieser Figur sind als weitere Größen noch die Höhe $h_F$ der durch die anteriore Schnittfläche 19 definierten Kugelkappe, die Höhe $h_L$ der durch die posteriore Schnittfläche 20 definierten Kugelkappe sowie die Dicke $d_L$ des zu entfernenden Volumens 18 eingezeichnet.

Die posteriore Schnittfläche 20 legt aufgrund des konstanten Abstandes zwischen Hornhautvorderfläche 15 und anteriorer Schnittfläche 19 den Krümmungsverlauf der Hornhautvorderfläche 15* nach Entfernung des Volumens 18 fest. Somit wird die posteriore Schnittfläche 20 z.B. bei einer zylindrische Parameter berücksichtigenden Fehlsichtigkeitskorrektur einen winkelabhängigen Krümmungsradius haben. Für die in Figur 7 gezeigte Lentikel-Fläche 20 gilt allgemein:

$$R_L(\varphi) = R_{CV}^*(\varphi) - d_F ,$$

bzw. in Zylinderkoordinaten (z, r, $\varphi$)

$$z_L(r, \varphi) = R_L(\varphi) - (R_L^2(\varphi) - r^2)^{1/2} + d_L + d_F.$$

Ohne Berücksichtigung eines Astigmatismus entfällt die Abhängigkeit von $\varphi$ und die Lentikel-Fläche 20 ist sphärisch. Die Lentikel-Fläche 20 besitzt aber, geht man vom Bedarf für eine zylindrische Fehlsichtigkeitskorrektur aus, in der Regel auf verschiedenen Achsen unterschiedliche Krümmungsradien, wobei diese natürlich meist den gleichen Scheitelpunkt haben.

Damit wird weiter automatisch deutlich, dass im Fall einer myopischen Zylinderkorrektur die theoretische Schnittlinie zwischen Flap-Fläche 19 und Lentikel-Fläche 20 nicht in einer Ebene, d.h. bei konstanten z-Koordinaten liegt. Der kleinste Krümmungsradius der Lentikel-Fläche 20 liegt bei $\varphi = \theta + \pi/2$, der größte natürlich auf der Achse $\theta$ der zylindrischen Fehlsichtigkeit, d.h. bei $\varphi = \theta$. Bei einer Übersichtigkeitskorrektur fallen anders bei der Darstellung der Figur 7 der Scheitelpunkt von Flap-Fläche 19 und Lentikel-Fläche 20 theoretisch zusammen und die Lentikel-Fläche 20 ist stärker gekrümmt, als die Flap-Fläche 19. Die Dicke $d_L$ des Lentikels ergibt sich in obiger Gleichung somit als zentrale Lentikeldicke bei Myopie. Das als Lentikel aufzufassende Volumen 18 hat im Falle der Myopiekorrektur am Rand theoretisch eine Schnittlinie von Lentikel-Fläche 20 und Flap-Fläche 19.

Bei Hyperopiekorrektur ist immer eine endliche Randdicke gegeben, da die Lentikel-Fläche 20 schwächer gekrümmt ist als die Flap-Fläche 19. Hier aber ist theoretisch die zentrale Lentikeldicke gleich Null. Deshalb ist neben der Flap-Fläche 20 und der Lentikel-Fläche 19 eine zusätzliche Randfläche vorgesehen, welche das von Flap-Fläche 20 und der Lentikel-Fläche 19 begrenzte Volumen 18 am Rand abschließt. Der Schnitt dieser Randfläche wird ebenfalls mit dem gepulsten Laserstrahl ausgeführt.

Die in den Figuren gezeigte Ausbildung des Volumens 18 als durch eine anteriore Schnittfläche 19 mit konstantem Abstand zur Hornhautvorderfläche 15 sowie eine posteriore Schnittfläche 20 begrenzt, ist nur eine Variante zur Begrenzung des Volumens 18. Sie hat jedoch den Vorteil, dass die optische Korrektur wesentlich nur durch eine Fläche (die Lentikelfläche 20) festgelegt wird, so dass die analytische Beschreibung der anderen Teilfläche der Grenzfläche einfach ist.

Weiter sind optimale Sicherheitsmargen hinsichtlich des Abstandes des Volumens zur Hornhautvorderfläche 15 und Hornhautrückfläche 16 gegeben. Die Restdicke $d_F$ zwischen anteriorer Schnittfläche 19 und Hornhautvorderfläche 15 kann konstant auf einen Wert von beispielsweise 50 bis 200 $\mu$m eingestellt werden. Insbesondere kann sie so gewählt sein, dass das schmerzempfindliche Epithel in der Lamelle verbleibt, die durch die Flap-Fläche 19 unter der Hornhautvorderfläche 15 gebildet ist. Auch steht die Ausbildung der sphärischen Flap-Fläche 19 in Kontinuität mit bisherigen Keratometerschnitten, was für die Akzeptanz der Methode vorteilhaft ist.

Nach Erzeugen der Schnittflächen 19 und 20 wird dann das derart isolierte Volumen 18 aus der Hornhaut 5 entfernt. Dies ist schematisch in Figur 8 dargestellt, die zudem verdeutlicht, dass die Schnittflächen 19 und 20 durch Einwirkung des in einem Fokuskegel 21 einfallenden Behandlungslaserstrahls erzeugt werden, beispielsweise durch Aneinanderreihung von Plasmablasen, so dass in einer bevorzugten Ausführungsform die Flap-Schnittfläche 19 und die Lentikel-Schnittfläche 20 durch geeignete dreidimensionale Verstellung der Fokuslage der gepulsten Laserstrahlung 2 erzeugt werden.

Alternativ kann in einer vereinfachten Ausführungsform aber auch lediglich die Flap-Fläche 19 durch Zielpunkte, die die gekrümmte Schnittfläche 19 in konstantem Abstand zu Hornhautvorderfläche 15 definieren mittels gepulster Laserstrahlung gebildet werden und die Entfernung des Volumens 18 erfolgt durch Laserablation, beispielsweise durch Verwendung eines Excimer-Laserstrahls. Hierzu kann die Lentikel-Fläche 20 als Grenzfläche des Abtrages definiert werden, auch wenn das nicht zwingend erforderlich ist. Das Behandlungsgerät 1 arbeitet dann wie ein bekanntes Laserkeratom, allerdings wird die Schnittfläche 19 an gekrümmter Hornhaut erzeugt. Die vorangehend bzw. nachfolgend beschriebenen Merkmale sind auch in solchen Varianten möglich, insbesondere was die Bestimmung der Begrenzungsfläche, deren geometrische Definition und die Ermittlung von Steuerparametern angeht.

Erzeugt man sowohl die Lentikel-Fläche 20 als auch die Flap-Fläche 19 mittels gepulster Laserstrahlung, ist es zweckmäßig, die Lentikel-Fläche 20 vor der Flap-Fläche 19 auszubilden, da das optische Ergebnis bei der Lentikel-Fläche 20 besser (wenn nicht überhaupt erst zu erreichen) ist, wenn oberhalb der Lentikel-Fläche 20 noch keine Veränderung der Hornhaut 5 eintrat.

Das Entfernen des durch die gepulste Laserstrahlung isolierten Volumens 18 kann, wie in Figur 8 angedeutet, durch einen Randschnitt 22 erreicht werden, der es erlaubt, das Volumen 18 in Richtung eines in Figur 8 eingezeichneten Pfeils 23 herauszuziehen. Alternativ kann der Randschnitt 22 aber so ausgebildet werden, dass er die anteriore Schnittfläche 19, d. h. die Flap-Fläche 19, in Form eines Ringes mit der Hornhautvorderfläche 15 verbindet, wobei der Randschnitt allerdings nicht vollständig um einen Winkel von 360° umläuft. Die derart isolierte Lamelle bleibt in einem schmale Bereich mit dem übrigen Gewebe der Hornhaut 5 in Verbindung. Diese Verbindungsbrücke dient dann als Gelenk, um die ansonsten isolierte Lamelle von der Hornhaut 5 abzuklappen und das dadurch zugängige, bereits isolierte Volumen 18 vom Rest der Augenhornhaut 5 abnehmen zu können. Die Lage der Verbindungsbrücke ist bei Erzeugung der Steuerdaten bzw. der Zielpunkte vorgebbar. Das beschriebene Vorgehen bzw. Gerät realisiert also unter diesem Gesichtspunkt die Isolierung des Volumens 19 innerhalb der Hornhaut 5 und das Erzeugen einer mit der restlichen Augenhornhaut über eine Gewebebrücke verbundenen Lamelle als Deckel über dem Volumen. Der Deckel kann abgeklappt und das Volumen 18 entnommen werden.

Für die Erzeugung der Schnittflächen 19 und 20 können die Zielpunkte nun auf verschiedenste Art und Weise angeordnet werden. Im Stand der Technik ist beispielsweise in der WO 2005/011546 zur Erzeugung von Schnittflächen in der Augenhornhaut beschrieben, dass spezielle Spiralen eingesetzt werden können, die beispielsweise um eine im wesentlichen senkrecht zur optischen Achse (z-Achse) liegende Hauptachse in Art einer Schraubenlinie verlaufen. Auch ist die Verwendung eines Scanmusters bekannt, das die Zielpunkte zeilenweise anordnet (vgl. WO 2005/011545). Diese Möglichkeiten können selbstverständlich zur Erzeugung der oben definierten Schnittflächen verwendet werden.

Die oben bereits erwähnte Randfläche ist in den Figuren 9a und 9b genauer zu sehen. Elemente, die bereits anhand anderer Figuren erläutert wurden, sind in diesen Figuren mit denselben Bezugszeichen versehen, so dass auf ihre Erläuterung gegebenenfalls verzichtet wird.

Figur 9a zeigt eine Draufsicht auf die Hornhautvorderfläche 15 mit der Flap-Fläche 19 sowie der Lentikel-Fläche 20 (deren Kante gestrichelt gezeichnet ist) sowie dem Randschnitt 22 im Falle einer Hyperopiekorrektur. Weiter ist in Figur 9a eine Übergangszone zwischen dem Rand der Lentikel-Fläche 20 und der Flap-Fläche 19 zu erkennen, die durch eine Randfläche 24 erreicht ist. Diese Randfläche 24 ist in Figur 9b deutlich zu sehen, die eine Schnittdarstellung durch die Darstellung der Figur 9a entlang der Linien A-A ist.

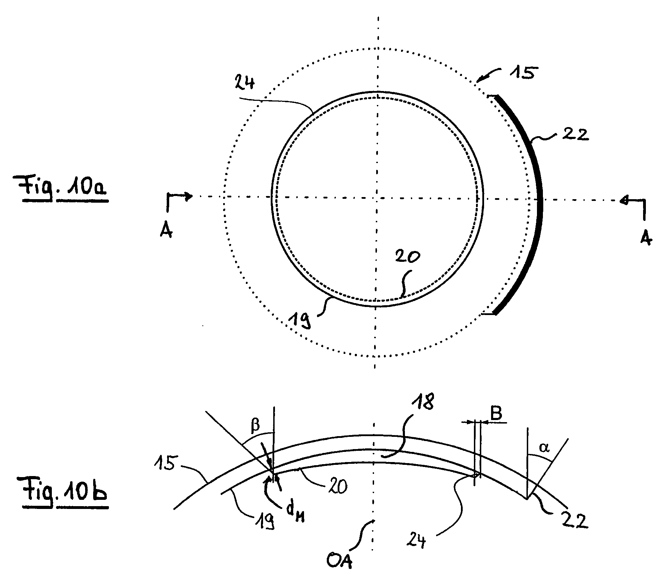

Das in der Figur 9b (und analog in den folgenden Figuren 10b, 11b sowie 12a) dargestellte Lentikel hat nun eine Mindestdicke, die im Falle eines Hyperopie korrigierenden Lentikels im Bereich der Sehachse OA, bei einer Myopie-Korrektur dagegen am Rand des Lentikels liegt. Die Mindestdicke $d_M$ bzw. $d_L$ gewährleistet, dass das zu entnehmende Gewebestück in Form des Lentikels 18 ausreichend stabil ist, so dass bei der Entnahme keine Partikel abreißen oder das Gewebestück zerbricht. Die Mindestdicke liegt vorzugsweise im Bereich von 5 bis 50 $\mu$m. Die Untergrenze ist dabei durch die Schichtdicke der Lamellenschichten gegeben, aus denen die Augenhornhaut aufgebaut ist. Unterschreitet man einen Wert von 5 $\mu$m nicht wesentlich, ist gewährleistet, dass das Lentikel an seiner dünnsten Stelle immer noch mindestens eine Lamellenschicht aufweist, wodurch die ausreichende Stabilität gegeben ist.

Die Randfläche 24 bewirkt den Übergang von der Lentikel-Fläche 20 zur Flap-Fläche 19. Sie ist dabei in der Ausführungsform der Figur 9b als konische Fläche ausgeführt, die schräger liegt, als eine (in Draufsicht auf Fig. 9a) konische Fläche, welche senkrecht zur Hornhautvorderfläche 15 oder zur parallel dazu verlaufenden Flap-Fläche 19 läge. Einen solchen Verlauf hat beispielsweise der Randschnitt 22, der bezogen auf die Seh-Achse OA in einem Winkel $\alpha$ liegt, der den erwähnten senkrechten Verlauf zur Hornhautvorderfläche zur Folge hat.

Die Randfläche 24 verläuft hingegen geneigter, so dass die Breite B, welche die Randfläche 24 bei Draufsicht in Richtung der optischen Achse OA hat, größer ist, als z. B. beim Randschnitt 24. Der entsprechende Winkel $\beta$ ist folglich ebenfalls größer als der Winkel $\alpha$.

Die Figuren 10a und 10b zeigen die entsprechenden Verhältnisse im Falle eines myopen Lentikels, d.h. bei der Myopie-Korrektur. Auch hier ist die Randfläche 24 vorhanden und führt zu einer endlichen Randdicke des Lentikels 18, welche an und für sich aufgrund der größeren Krümmung der Lentikel-Fläche 20 gegenüber der Flap-Fläche 10 nicht gegeben wäre, da beide Flächen zumindest in ihrer Fortsetzung eine Schnittlinie haben würden, also der Rand in einer Schnittlinie endete.

Die Mindestdicke des Lentikels 18, welche bei einer hyperopen Form gem. Figur 9b an oder nahe der optischen Achse OA vorliegt, besteht bei einem myopen Lentikel gem. Figur 10b also am Rand. Entsprechend ist die Mindestdicke $d_M$ in Fig. 10b auch am Rand eingezeichnet. Auch hier bewirkt die Mindestdicke, daß das Lentikel bei der Entnahme ausreichend stabil ist.

Selbstverständlich können die Schnittdarstellungen der Figuren 9b und 10b nur dann beschreibend für das gesamte Lentikel sein, wenn keine Korrektur höherer Ordnung, insbesondere kein Astigmatismus, vorliegt. Ist solches gegeben, ist die Lentikelfläche 20 von Sphärizität abweichend entsprechend korrigiert, was sich auf für den Fachmann naheliegende Weise auch auf die Randfläche 24 auswirkt.

Die Figuren 11a und 11b zeigen schließlich Verhältnisse entsprechend denen der Figuren 9a und 9b, jedoch verläuft hier der Randschnitt 22, welcher zur Freilegung des Lentikels 18 erzeugt wird, über einen sehr viel größeren Winkelbereich, als in Figuren 9 und 10. Wie bei den Figuren 9a und 9b ist die Mindestdicke auch hier im Bereich der Sehachse gegeben, da das Lentikel hyperop ist.

Die Figuren 9 bis 11 zeigen eine Randfläche 24, die als schräger Schnitt ausgeführt ist. Die Randfläche 24 kann jedoch auch eine von einem solchen, in Querschnittansicht geradlinigen Verlauf abweichende Struktur haben. Dies ist exemplarisch in den Figuren 12a bis d gezeigt. Hierin gibt Figur 12a eine Schnittdarstellung ähnlich der der Figuren 9b, 10b oder 11b wieder. Die Figuren 12b bis 12d zeigen den in der Figur 12a punktierten Ausschnitt vergrößert und stellen unterschiedliche Varianten für die Struktur der Randfläche 24 dar.

Gemäß Figur 12b besteht die Randfläche 24 aus zwei im Querschnitt im wesentlichen geradlinigen Abschnitten 25 und 26. Der erste Abschnitt 25 läuft senkrecht in die Flap-Fläche 19 ein. Die Höhe des ersten Abschnittes 25 bewirkt eine Dicke $d_R$ des Randes. Diese Dicke ist vorzugsweise im Bereich von 5 bis 10 μm gewählt und garantiert, dass beim Entnehmen des Lentikels 18 kein Abreißen von Bruchstücken im Bereich der Randfläche 24 auftritt. Solche Bruchstücke hätten erheblich nachteilige Auswirkungen auf das Einwachsen und würden zu einer unerwünschten Regression führen. Um trotz des senkrecht zur Flap-Fläche 19 liegenden ersten Abschnittes 25 ein sanftes Anlegen der durch den Flap-Schnitt 19 isolierten Hornhautlamelle 27 zu erreichen, wenn das lentikelförmige Volumen entnommen wurde, hat der zweite Abschnitt 26 der Randfläche 24 in der Ausführungsform der Figur 12b einen schrägen Verlauf zur Seh-Achse hin. Durch diesen schrägen Verlauf ist wiederum eine Breite B erreicht, die deutlich größer als bei einem durchgängig senkrecht zur Flap-Fläche 19 verlaufenden Rand 24 gegeben wäre.

Figur 12c zeigt eine Abwandlung der Struktur der Randfläche 24 der Figur 12b, bei der sich an den senkrecht in die Flap-Fläche 19 einlaufenden ersten Abschnitt 25 ein kontinuierlich gekrümmter zweiter Abschnitt 26 ausgebildet ist, durch den der Rand zwischen der Lentikel-Fläche 20 und der Flap-Fläche 19 im Bereich, der von der Augenhornhautvorderfläche 15 abgewandt ist, abgerundet ist. Das senkrechte Einlaufen des ersten Abschnittes 25 stellt wiederum eine Mindestranddicke $d_R$ sicher, die ein Abreißen von Gewebestücken am Lentikelrand bei der Entnahme des Lentikelgewebes verhindert. Vorzugsweise ist die Mindestranddicke in gleicher Größenordnung wie die Mittendicke $d_M$.

Dies ist auch bei der Randstruktur gem. Figur 12d erreicht, die S-förmig ausgebildet ist, wobei wiederum der erste Abschnitt 25 senkrecht in die Flap-Fläche 19 einläuft. Aufgrund der S-Struktur weist die Schnittdarstellung der Randfläche 24 in dieser Ausgestaltung einen Wendepunkt auf, und der zweite Abschnitt 26 endet vorzugsweise ebenfalls rechtwinklig in der Lentikel-Fläche 20.

Das Kontaktglas hat den weiteren Vorteil, dass durch das Anpressen an die sphärische Kontaktglasunterseite 26 automatisch auch die Hornhautvorderfläche 15 sphärisch ist. Die in konstantem Abstand unter der Hornhautvorderfläche 15 liegende anteriore Schnittfläche 19 ist damit bei angepresstem Kontaktglas ebenfalls sphärisch, was zu erheblich vereinfachter Ansteuerung führt. Es ist deshalb völlig unabhängig von anderen Merkmalen bevorzugt, ein Kontaktglas mit sphärischer Kontaktglasunterseite zu verwenden und das Volumen durch eine anteriore Schnittfläche 19 sowie eine posteriore Schnittfläche zu begrenzen, wobei die anteriore Schnittfläche als sphärische Fläche in konstantem Abstand $d_F$ unter der Hornhautvorderfläche 15 erzeugt wird. Die posteriore Schnittfläche hat einen Krümmungsverlauf, welcher bei relaxiertem Auge, also nach Abnehmen des Kontaktglases, bis auf den Abstand $d_F$ zur Hornhautvorderfläche dem zur Fehlsichtigkeitskorrektur gewünschten entspricht. Analoges gilt für die Definition der Zielpunkte bzw. das Operationsverfahren.

**Patentansprüche**

1. Behandlungsvorrichtung zur operativen Myopie- oder Hyperopie-Korrektur am Auge (3), wobei die Behandlungsvorrichtung (1) eine von einer Steuereinrichtung (12) gesteuerte Lasereinrichtung (L) aufweist, welche durch Einstrahlen von Laserstrahlung (2) Hornhaut-Gewebe trennt, wobei die Steuereinrichtung (12) ausgebildet ist, die Lasereinrichtung (L) zur Abgabe der Laserstrahlung (2) in die Hornhaut (5) so anzusteuern, dass damit ein lentikelförmiges Volumen (18) in der Hornhaut (5) isoliert ist, dessen Entfernung aus der Hornhaut (18) die gewünschte Korrektur bewirkt, **dadurch gekennzeichnet, dass** die Steuereinrichtung (12) bei der Ansteuerung der Lasereinrichtung (L) das lentikelförmige Volumen (18) so vorgibt, dass es eine Mindestdicke ($d_M$) im Bereich von 5 bis 50 μm hat, wobei bei Myopie-Korrektur die Mindestdicke ($d_M$) am Rand des Volumens (18) und bei Hyperopie-Korrektur im Bereich der Sehachse (OA) vorliegt.

2. Verfahren zum Erzeugen von Steuerdaten für eine Lasereinrichtung (L) einer Behandlungsvorrichtung (1) zur operativen Myopie- oder Hyperopie-Korrektur am Auge (3), welche durch Einstrahlen von Laserstrahlung (2) Hornhaut-Gewebe trennt, wobei die Steuerdaten im Betrieb die Lasereinrichtung (L) zur Abgabe der Laserstrahlung (2) in die Hornhaut (5) so ansteuern, dass damit ein lentikelförmiges Volumen (18) in der Hornhaut (5) isoliert ist, dessen Entfernung aus der Hornhaut (18) die gewünschte Korrektur bewirkt, **dadurch gekennzeichnet, dass** die Steuerdaten das lentikelförmige Volumen (18) so vorgeben, dass es eine Mindestdicke ($d_M$) im Bereich von 5 bis 50 μm hat, wobei bei Myopie-Korrektur die Mindestdicke ($d_M$) am Rand des Volumens (18) und bei Hyperopie-Korrektur im Bereich der Sehachse (OA) vorliegt.

3. Vorrichtung oder Verfahren nach einem der obigen Ansprüche, **dadurch gekennzeichnet, dass** das lentikelförmige Volumen eine anteriore Fläche (19) und eine posteriore Fläche (20) hat, wobei die anteriore Fläche (19) in einem konstantem Abstand $d_F$ zur Hornhautvorderfläche (15) liegt und die posteriore Fläche gekrümmt ist und einen Krümmungsradius $R_L = R_{CV}{}^* - d_F$ hat, wobei zur Bestimmung der Steuerdaten folgender Zusammenhang verwendet wird:

$$R_{CV}^* = 1 / ( (1/R_{CV}) + B_{BR} / ( (n_c\text{-}1) \bullet (1 - d_{HS} \bullet B_{BR}))) + F,$$

und $R_{CV}$ der Krümmungsradius der Hornhaut (5) vor Entfernung des Volumens (18), $n_c$ die Brechkraft des Materials der Hornhaut (5), F ein Korrektur-Faktor ist, $B_{BR}$ die Brechkraft einer für die Fehlsichtigkeitskorrektur tauglichen Brille (17), sowie $d_{HS}$ der Abstand ist, in dem die Brille (17) mit der Brechkraft $B_{BR}$ vor dem Hornhautscheitel liegen müsste, um die gewünschte Fehlsichtigkeitskorrektur mittels der Brille (17) zu erreichen.

4. Vorrichtung oder Verfahren nach Anspruch 3, **dadurch gekennzeichnet, dass**

$$F = (1 - 1/n_c) \bullet (d_C^* - d_C)$$

gilt, wobei $d_C$ bzw. $d_C^*$ die Dicke der Hornhaut (5, 5*) vor bzw. nach Entfernung des Volumens (18) bezeichnet und der Radius $R_{CV}^*$ iterativ berechenbar ist, indem bei jedem Iterationsschritt aus der Differenz $(R_{CV}^* - R_{CV})$ auf eine Dickenänderung $(d_C^* - d_C)$ geschlossen wird und das entsprechende daraus erhaltene Ergebnis für die Dickenänderung bei der Berechnung von $R_{CV}^*$ im nächsten Iterationsschritt angewendet wird.

5. Vorrichtung oder Verfahren nach Anspruch 3, **dadurch gekennzeichnet, dass** die posteriore Fläche (20) in Zylinderkoordinaten (z, r, φ), deren Ursprung am Durchtrittspunkt der Sehachse (OA) durch die Hornhautvorderfläche (15) liegt, der Gleichung

$$z_L(r,φ) = R_L(φ) - (R_L^2(φ) - r^2)^{1/2} + d_M + d_F$$

genügt.

6. Vorrichtung oder Verfahren nach einem der obigen Ansprüche, **dadurch gekennzeichnet, dass** das lentikelförmige Volumen eine posteriore Fläche und eine anteriore Fläche hat, deren Ränder über eine Randfläche verbunden sind, wobei die Schnittkurve aus der Randfläche und einer Ebene, in der die Sehachse enthalten ist, senkrecht zur Sehachse eine Breite hat, die größer als diejenige ist, welche in der gleichen Projektionsebene eine gerade Strecke hätte, die am Rand der posterioren oder der anterioren Fläche auf der jeweiligen Fläche senkrecht steht und die anteriore Fläche mit der posterioren Fläche oder mit deren gedachter Fortsetzung verbindet.

7. Vorrichtung oder Verfahren nach Anspruch 6, **dadurch gekennzeichnet, dass** die Randfläche (24) einen ersten, weitgehend senkrecht in die anteriore Fläche mündenden Abschnitt (25) und einen zweiten, stärker zur Sehachse (OA) hin geneigten Abschnitt (26) aufweist.

8. Vorrichtung oder Verfahren nach Anspruch 7, **dadurch gekennzeichnet, dass** der erste Abschnitt eine Höhe $d_R$ von mehr als 5 μm, insbesondere von mindestens 10 μm hat.

9. Vorrichtung oder Verfahren nach Anspruch 7 oder 8, **dadurch gekennzeichnet, dass** der zweite Abschnitt (26) zur Richtung der Sehachse (OA) in einem Winkel von 80° bis 100° liegt.

10. Vorrichtung oder Verfahren nach den Anspruch 7, **dadurch gekennzeichnet, dass** der zweite Abschnitt (26) konkav ist, bezogen auf den Durchtrittspunkt der Sehachse (OA) durch die Hornhautvorderfläche (15).

11. Vorrichtung oder Verfahren nach Anspruch 7, **dadurch gekennzeichnet, dass** bei einer Hyperopiekorrektur der erste Abschnitt eine Höhe $d_R$ hat, die der Mindestdicke $d_M$ im Rahmen der Schnittflächegenauigkeit gleicht.

**Claims**

1. Treatment apparatus for operatively correcting myopia or hyperopia in an eye (3), the treatment apparatus (1) comprising a laser device (L) which is controlled by a control device (12) and separates corneal tissue by applying laser radiation (2), the control device (12) being adapted to control the laser device (L) for emitting the laser radiation (2) into the cornea (5) such that a lenticular volume (18) is thus isolated in the cornea (5), the removal of which volume from the cornea (5) effects the desired correction, **characterized in that** the control device (12) defines the lenticular volume (18) when controlling the laser device (L) such that the lenticular volume has a minimum thickness ($d_M$) in the range of from 5 to 50 $\mu$m, the minimum thickness ($d_M$) being at the edge of the volume (18) in correction of myopia and in the region of the axis of vision (OA) in correction of hyperopia.

2. Method for generating control data for a laser device (L) of a treatment apparatus (1) for operatively correcting myopia or hyperopia in the eye (3), which laser device separates corneal tissue by applying laser radiation (2), wherein the control data control operation of the laser device (L) for emitting the laser radiation (2) into the cornea (5) in such a way that a lenticular volume (18) is thus isolated in the cornea (5), the removal of which volume from the cornea (5) effects the desired correction, **characterized in that** the control data define the lenticular volume (18) such that it has a minimum thickness ($d_M$) in the range of from 5 to 50 $\mu$m, the minimum thickness ($d_M$) being at the edge of the volume (18) in correction of myopia and in the region of the axis of vision (OA) in correction of hyperopia.

3. Apparatus or method according to one of the above claims, **characterized in that** the lenticular volume comprises an anterior face (19) and a posterior face (20), wherein the anterior face (19) is positioned at a constant distance $d_F$ from the front face (15) of the cornea and the posterior face is curved and has a radius of curvature $R_L = R_{CV}{}^* - d_F$, wherein the following equation is used to determine the control data

$$R_{CV}{}^* = 1 / (\, (1/R_{CV}) + B_{BR} / (\, (n_c\text{-}1) \cdot (1 - d_{HS} \cdot B_{BR}))) + F,$$

and wherein $R_{CV}$ is the radius of curvature of the cornea (5) before removal of the volume (18), $n_c$ is the refractive power of the material of the cornea (5), F is a correction factor, $B_{BR}$ is the refractive power of a pair of spectacles (17) suitable for correcting the defective vision, and $d_{HS}$ is the distance at which the pair of spectacles (17) having the refractive power $B_{BR}$ would have to be positioned before the corneal vertex in order to achieve the desired correction of defective vision by means of the pair of spectacles (17).

4. Apparatus or method according to claim 3, **characterized in that**

$$F = (1 - 1/n_c) \cdot (d_C{}^* - d_C),$$

applies, wherein $d_C$ and $d_C{}^*$ denote the thickness of the cornea (5, 5*) before and after removal of the volume (18), respectively, and the radius $R_{CV}{}^*$ can be calculated iteratively **in that** during each iteration step a change in thickness ($d_C{}^* - d_C$) is derived from the differential ($R_{CV}{}^* - R_{CV}$) and the corresponding result obtained therefrom for the change in thickness is applied in the calculation of $R_{CV}{}^*$ in the next iteration step.

5. Apparatus or method according to claim 3, **characterized in that** the posterior face (20) in cylindrical coordinates (z, r, $\varphi$), the origin of which is positioned at the point at which the axis of vision (OA) passes through the front face (15) of the cornea, satisfies the equation

$$z_L(r, \varphi) = R_L(\varphi) - (R_L{}^2(\varphi) - r^2)^{1/2} + d_M + d_F.$$

6. Apparatus or method according to one of the above claims, **characterized in that** the lenticular volume has a posterior face and an anterior face, the edges of which are connected via an edge face, the cut curve made up of the edge face and a plane which comprises the axis of vision has - measured perpendicular to the axis of vision - a width which is greater than that of a straight line which is perpendicular to the same projection plane and at the edge of the posterior or the anterior face on the respective face and which connects the anterior face to the posterior face or to a notional continuation thereof.

7. Apparatus or method according to claim 6, **characterized in that** the edge face (24) has a first portion (25) opening substantially perpendicularly into the anterior face and a second portion (26) more inclined toward the axis of vision (OA).

8. Apparatus or method according to claim 7, **characterized in that** the first portion has a height $d_R$ of more than 5

μm, in particular of at least 10 μm.

9. Apparatus or method according to claim 7 or 8, **characterized in that** the second portion (26) is positioned at an angle of from 80o to 100o relative to the direction of the axis of vision (OA).

10. Apparatus or method according to claim 7, **characterized in that** the second portion (26) is concave in relation to the point at which the axis of vision (OA) passes through the front face (15) of the cornea.

11. Apparatus or method according to claim 7, **characterized in that** in a correction of hyperopia the first portion has a height $d_R$ which is equal to the minimum thickness $d_M$ within the limits of cut face accuracy.

**Revendications**

1. Dispositif de traitement permettant la correction chirurgicale de la myopie ou de l'hyperopie d'un oeil (3), dans lequel le dispositif de traitement (1) présente un dispositif laser (L) commandé par un dispositif de commande (12), qui sépare du tissu cornéen par irradiation avec un rayonnement laser (2), dans lequel le dispositif de commande (12) est conçu pour commander le dispositif laser (L) en vue de l'émission du rayonnement laser (2) dans la cornée (5) de telle manière qu'un volume lenticulaire (18) soit ainsi isolé dans la cornée (5), dont l'enlèvement hors de la cornée (18) provoque la correction désirée, **caractérisé en ce que** le dispositif de commande (12) prescrit le volume lenticulaire (18) lors de la commande du dispositif laser (L) de telle manière qu'il présente une épaisseur minimale ($d_M$) dans la plage de 5 à 50 μm, dans lequel en cas de correction de myopie l'épaisseur minimale ($d_M$) se trouve sur le bord du volume (18) et en cas de correction d'hyperopie elle se trouve dans la région de l'axe de vision (OA).

2. Procédé de génération de données de commande pour un dispositif laser (L) d'un dispositif de traitement (1) permettant la correction chirurgicale de la myopie ou de l'hyperopie dans l'oeil (3), qui sépare du tissu cornéen par irradiation avec un rayonnement laser (2), dans lequel les données de commande commandent en service le dispositif laser (L) en vue de l'émission du rayonnement laser (2) dans la cornée (5) de telle manière qu'un volume lenticulaire (18) soit ainsi isolé dans la cornée (5), dont l'enlèvement hors de la cornée (18) provoque la correction désirée, **caractérisé en ce que** les données de commande prescrivent le volume lenticulaire (18) de telle manière qu'il présente une épaisseur minimale ($d_M$) dans la plage de 5 à 50 μm, dans lequel en cas de correction de myopie l'épaisseur minimale ($d_M$) se trouve sur le bord du volume (18) et en cas de correction d'hyperopie elle se trouve dans la région de l'axe de vision (OA).

3. Dispositif ou procédé selon l'une des revendications précédentes, **caractérisé en ce que** le volume lenticulaire comporte une face antérieure (19) et une face postérieure (20), dans lequel la face antérieure (19) se trouve à une distance constante $d_F$ de la face avant de la cornée (15) et la face postérieure est courbe et a un rayon de courbure $R_L = R_{CV}^* - d_F$, dans lequel, pour la détermination des données de commande, on utilise la relation suivante:

$$R_{CV}^* = 1/((1/R_{CV}) + B_{BR}/((n_c - 1)\cdot(1-d_{HS}\cdot B_{BR}))) + F,$$

et Rcv est le rayon de courbure de la cornée (5) avant l'enlèvement du volume (18), $n_c$ est la réfringence du matériau de la cornée (5), F est un facteur de correction, $B_{BR}$ est la réfringence d'un verre de lunette (17) convenant pour la correction de l'amétropie, et $d_{HS}$ est la distance à laquelle le verre de lunette (17) avec la réfringence $B_{BR}$ devrait se trouver devant le sommet de la cornée, pour obtenir la correction d'amétropie désirée avec le verre de lunette (17).

4. Dispositif ou procédé selon la revendication 3, **caractérisé en ce que** l'on utilise

$$F = (1-1/n_c)\cdot(d_c^* -d_c),$$

dans laquelle $d_c$ ou $d_{c*}$ désigne l'épaisseur de la cornée (5, 5*) avant ou après l'enlèvement du volume (18) et le rayon $R_{CV}^*$ peut être calculé de façon itérative, du fait qu'à chaque pas d'itération on déduit une variation d'épaisseur ($d_c^* - d_c$) à partir de la différence ($R_{CV}^* - R_{CV}$) et on utilise le résultat correspondant ainsi obtenu pour la variation d'épaisseur lors du calcul de $R_{CV}^*$ dans le pas d'itération suivant.

**5.** Dispositif ou procédé selon la revendication 3, **caractérisé en ce que** la face postérieure (20) répond à l'équation

$$z_L(r, \varphi) = R_L(\varphi) - (R_L^2(\varphi) - r^2)^{\frac{1}{2}} + d_M + d_F$$

en coordonnées cylindriques (z, r, $\varphi$), dont l'origine est située au point de percée de l'axe de vision (OA) dans la face avant de la cornée (15).

**6.** Dispositif ou procédé selon l'une quelconque des revendications précédentes, **caractérisé en ce que** le volume lenticulaire comporte une face postérieure et une face antérieure, dont les bords sont reliés par une face latérale, dans lequel la courbe d'intersection entre la face latérale et un plan, dans lequel l'axe de vision est contenu, présente perpendiculairement à l'axe de vision une largeur qui est plus grande que celle qu'aurait dans le même plan de projection un segment droit qui, sur le bord de la face postérieure ou de la face antérieure, est perpendiculaire à la face respective et qui relie la face antérieure à la face postérieure ou à son prolongement virtuel.

**7.** Dispositif ou procédé selon la revendication 6, **caractérisé en ce que** la face latérale (24) présente une première section (25) débouchant largement perpendiculairement dans la face antérieure et une deuxième section (26) plus fortement inclinée par rapport à l'axe de vision (OA).

**8.** Dispositif ou procédé selon la revendication 7, **caractérisé en ce que** la première section a une hauteur $d_R$ de plus de 5 $\mu$m, en particulier d'au moins 10 $\mu$m.

**9.** Dispositif ou procédé selon la revendication 7 ou 8, **caractérisé en ce que** la deuxième section (26) est située sous un angle de 80° à 100° par rapport à la direction de l'axe de vision (OA).

**10.** Dispositif ou procédé selon la revendication 7, **caractérisé en ce que** la deuxième section (26) est concave par rapport au point de percée de l'axe de vision (OA) dans la face avant de la cornée (15).

**11.** Dispositif ou procédé selon la revendication 7, **caractérisé en ce que** pour une correction d'hyperopie, la première section a une hauteur $d_R$, qui est égale à l'épaisseur minimale du dans le cadre de la précision de la face de coupe.

FIG 1

FIG 2

Fig. 5

Fig. 6

Fig. 1a

FIG 3

Fig. 7

Fig.4

a)

b)

c)

FIG 8

Fig. 12 b

Fig. 12 a

Fig. 12 c

Fig. 12 d

Fig.11a

Fig.11b

Fig. 10a

Fig. 10b

EP 2 211 804 B1

Fig. 9a

Fig. 9b

24

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2004243112 A1
- US 6110166 A
- US 6325792 B1
- US 2006155265 A1
- US 2003212387 A1
- DE 102005014760 A1
- DE 102005049281 A1
- US 5984916 A
- EP 1159986 A1
- US 5549632 A
- DE 69500997 T2
- WO 2004032810 A2
- WO 2005011545 A